Europäisches Patentamt

European Patent Office

Office européen des brevets

(19)

(11) Veröffentlichungsnummer: **0 000 028**
**B1**

(12) **EUROPÄISCHE PATENTSCHRIFT**

(45) Veröffentlichungstag der Patentschrift: **22.04.81**

(21) Anmeldenummer: **78100045.0**

(22) Anmeldetag: **01.06.78**

(51) Int. Cl.³: **C 12 N 11/14,** C 12 N 9/02,
C 12 N 9/92

(54) **Verfahren zur Herstellung eines wasserunlöslichen Enzympräparats.**

(30) Priorität: **10.06.77 DE 2726188**

(43) Veröffentlichungstag der Anmeldung:
**20.12.78 Patentblatt 78/1**

(45) Bekanntmachung des Hinweises auf die Patenterteilung:
**22.04.81 Patentblatt 81/16**

(84) Benannte Vertragsstaaten:
**BE CH DE FR GB LU NL SE**

(56) Entgegenhaltungen:
**FR - A - 2 020 527
FR - A - 2 223 324
FR - A - 2 273 008**

(73) Patentinhaber: **Kali-Chemie Aktiengesellschaft
Postfach 220 Hans-Böckler-Allee 20
D-3000 Hannover 1 (DE)**

(72) Erfinder: **Weidenbach, Günter, Dipl.-Chem.
Dr.rer.nat.
Hannoversche Strasse 52
D-3000 Hannover 73 (DE)
Erfinder: Bonse, Dirk, Dipl.-Chem. Dr.-Ing.
Berliner Ring 3
D-3161 Arpke (DE)**

Courier Press, Leamington Spa, England.

# 0 000 028

## Verfahren zur Herstellung eines wasserunlöslichen Enzympräparats

Es ist bekannt, Enzyme durch Bindung an organische oder anorganische Träger zu immobilisieren, d.h. wasserunlöslich zu machen, so dass diese wiederverwendbar sind und in kontinuierlich arbeitenden Verfahren eingesetzt werden können.

Organische Materialien (z.B. Zellulose, Nylon, Polyacrylamid) weisen als Träger erhebliche Nachteile auf, da sie keine ausreichende mechanische Stabilität besitzen, vom Lösungsmittel angegriffen werden können, gegenüber wechselnden pH-Werten und Ionenstärken empfindlich sind und teilweise zum Mikrobenbefall neigen, wodurch die Bindung zum Enzym gelöst werden kann.

Deshalb wurden anorganische Substanzen als Träger vorgeschlagen, auf denen Enzyme adsorptiv oder kovalent gebunden werden. Die bevorzugte Art der Bindung hängt von der Art und den Einsatzbedingungen des Enzyms und der Beschaffenheit des Substrates ab. Liegt das Substrat beispielsweise in einer starken Salzkonzentration vor, ist die Adsorptionsmethode nicht anwendbar, da eine Desorption der adsorbierten Enzymmoleküle möglich ist. Deshalb wird die kovalente Bindung des Enzyms an den Träger vorgezogen. Die Trägeroberfläche muss dann spezifische funktionelle Gruppen enthalten, die eine Bindung des Enzyms gewährleisten. Da der Träger diese funktionellen Gruppen in den meisten Fällen nicht besitzt, ist eine Vorbehandlung der Oberfläche erforderlich. Bekannt ist beispielsweise die Belegung von anorganischem Material mit Silanen, wodurch die Oberfläche organisch funktionelle Gruppen (z.B. Alkylamin) erhält, die mit organischer Substanz eine kovalente Bindung eingehen. Weiterhin ist die Behandlung anorganischer Träger mit Sulfurylchlorid, Thionylchlorid oder Cyanurchlorid erprobt worden. Ausserdem ist es möglich, die Trägeroberfläche mit einem wasserunlöslichen Polymer zu überziehen, das freie funktionelle Gruppen aufweist, wie z.B. Polyacrolein, das zwischen 10 und 70% freie Aldehydgruppen, bezogen auf die Anzahl der monomeren Moleküle, besitzt.

Als Material für anorganischeTräger wurden Aluminiumoxide, Nickeloxid, Eisenoxid, Titanoxid, Zirkonoxid, Hydroxylapatit, Silikate und poröses Glas vorgeschlagen, deren Porenstruktur die Zugänglichkeit des Enzyms und des Substrates zur inneren Oberfläche gewährleistet, über deren weitere wünschenswerte Eigenschaften wie optimale Porenverteilung und Oberflächengrösse jedoch sehr unterschiedliche Angaben vorliegen.

Mit keinem der genannten Träger gelang es, unabhängig von der angewandten Bindungsart, Enzyme so zu immobilisieren, dass ihre spezifische Aktivität der spezifischen Aktivität im freien Zustand nahekommt. Nach Angaben von D.L.Latigue (Immobilized Enzymes for Industrial Reactors, London 1975, S. 127) liegen auch unter besten Immobilisierungsbedingungen nur maximal 80% des auf dem Träger aufgebrachten Enzyms in aktiver Form vor.

Der Erfindung liegt daher die Aufgabe zugrunde, die bekannten Verfahren zur Herstellung eines wasserunlöslichen Enzympräparats, bei dem man einen anorganischen Träger, der funktionelle Gruppen zur kovalenten Bindung eines Enzyms aufweist, mit einer Lösung eines Enzyms in Berührung bringt, das Enzym mittels an sich bekannter Verfahren an den Träger bindet und die erhaltenen Enzympräparate isoliert, so zu verbessern, daß bei minimalem Enzymaufwand ein maximal aktives Präparat erhalten wird.

Die Lösung dieser Aufgabe gelingt gemäß der Erfindung dadurch, daß man zunächst Träger, die sich in ihrem häufigsten Porendurchmesser unterscheiden, mit Enzymlösungen, die sich in ihrer Enzymkonzentration unterscheiden, in Berührung bringt, das Enzym an die Träger bindet, die Enzympräparate isoliert, deren Aktivität bestimmt und von den benutzten unterschiedlichen Trägern den Träger mit demjenigen häufigsten Porendurchmesser auswählt, der ungeachtet der an ihn gebundenen Enzymmenge das Präparat mit der höchsten Aktivität ergeben hat, und dann den ausgewählten Träger mit Enzymlösungen, die sich in ihrem Enzymgehalt unterscheiden, in Berührung bringt, das Enzym an den Träger bindet, die Enzympräparate isoliert, deren Aktivität und spezifische Aktivität bestimmt und von den benutzten unterschiedlichen Enzymlösungen diejenige auswählt, die das Präparat mit der höchsten Aktivität und einer spezifischen Aktivität nahe oder gleich der spezifischen Aktivität des Enzyms im freien Zustand ergeben hat.

Bei der Lehre nach der Erfindung werden zunächst Träger mit unterschiedlichen häufigsten Porendurchmessern nach bekannten Methoden mit Kupplungsmitteln versehen, die sowohl ausreichend fest am Träger haften, insbesondere eine kovalente Bindung zum Träger eingehen, als auch zu einer kovalenten Bindung mit dem Enzym befähigt sind. Als gebräuchlichste Methode hat sich in der Vergangenheit für anorganische Träger die Silanisierung durchgesetzt, es sind aber, wie schon ausgeführt, auch andere Kupplungsmittel einsetzbar. Die Anzahl der Kupplungsglieder am Träger muss hinreichend gross sein und hängt weitgehend von der Oberfläche des Trägers ab.

Den so vorbehandelten Trägern werden dann unterschiedliche Enzymmengen angeboten, indem man sie mit Enzymlösungen unterschiedlicher Konzentration in Berührung bringt und nach bekannten Methoden eine kovalente Bindung des Enzyms zum Kupplungsglied und damit zum Träger herstellt. Nach Bestimmung der Aktivität der so erhaltenen Präparate zeigt sich, dass die Aktivität der Präparate ungeachtet der an sie gebundenen Enzymmenge von dem häufigsten Porendurchmesser abhängt und ein Maximum durchläuft. Es hat sich dabei ausserdem gezeigt, dass die Korngrösse des Trägers für die Lage des Maximums unerheblich ist und allenfalls dessen Absolutwert beeinflusst. Die Korngrösse des Trägers hat daher für die Lehre nach der Erfindung nur eine untergeordnete Bedeutung und richtet sich weitgehend nach dem vorgesehenen Einsatzzweck, beispielsweise der Viskosität des Substrats und der Verfahrensführung.

Dem auf diese Weise hinsichtlich des häufigsten Porendurchmessers ermittelten optimalen Träger werden dann wiederum unterschiedliche Mengen Enzym angeboten. Dabei zeigt sich, dass bei bestimmten Enzymkonzentrationen Präparate erhalten werden, deren spezifische Aktivität der spezifischen Aktivität des Enzyms im freien Zustand nahekommt oder diese erreicht, d.h. die relative Aktivität des Präparats erreicht einen Wert von 100%.

Bei dem erfindungsgemäßen Verfahren handelt es sich also um ein zweifaches Auswahlverfahren, d.h. um ein Verfahren, mit dessen Hilfe man aus mehreren hinsichtlich ihres Porendurchmessers in Betracht kommenden Trägern, und aus mehreren, hinsichtlich ihrer Enzymkonzentration in Betracht kommenden Enzymlösungen, denjenigen Träger und diejenige Enzymkonzentration auswählt, die ein Präparat mit höchstmöglicher Aktivität bei geringstmöglichem Enzymeinsatz ergeben.

Wenn man nämlich erfindungsgemäß mehrere, hinsichtlich ihres häufigsten Porendurchmessers unterschiedliche Träger je für sich mit einer Enzymlösung mit einer bestimmten, zunächst für alle Träger gleichen Konzentration umsetzt, danach diese Operation mit denselben Trägern jedoch mit Enzymlösungen mit von der erstgenannten Enzymlösung verschiedener Konzentration wiederholt, von den erhaltenen Enzympräparaten die absolute Aktivität, d.i. die Aktivität pro Gramm Enzympräparat, bestimmt und die Aktivitäten miteinander vergleicht, dann zeigt sich, daß unabhängig von der Konzentration der benutzten Enzymlösungen immer der hinsichtlich seines häufigsten Porendurchmessers gleiche Träger das aktivste Präparat ergeben hat.

Es zeigt sich aber auch, im Gegensatz zu dem zu erwartenden Ergebnis, daß nämlich mit steigender Konzentration der Enzymlösungen auch die Aktivität der erhaltenen Enzympräparate steigen sollte, von einer bestimmten Konzentration der Enzymlösung an eine weitere Steigerung der Konzentration keinen Aktivitätszuwachs mehr bringt, d.h. ein "Mehr" an Enzym bringt nicht unbedingt auch ein "Mehr" an Aktivität.

Das wird besonders augenscheinlich, wenn man gemäß der Erfindung den ermittelten optimalen Träger mit unterschiedlich konzentrierten Enzymlösungen umsetzt, von den erhaltenen Enzympräparaten die absolute Aktivität und die spezifische Aktivität, d.i. die Aktivität pro Milligramm Enzym, bestimmt, und die erhaltenen Werte miteinander vergleicht. Dabei bestätigt sich zunächst, daß mit steigender Konzentration der Enzymlösung die absolute Aktivität der erhaltenen Präparate zunächst ebenfalls steigt, dann aber von einer bestimmten Konzentration an gleich bleibt. Jede Steigerung der Konzentration über die so ermittelte Konzentration hinaus ist also Enzymverschwendung. Das steht im Widerspruch zu den bekannten Erfahrungen, die nach dem Motto handeln "Viel hilft Viel".

Es zeigt sich aber auch, daß bei niedrigen Konzentrationen der benutzten Enzymlösungen das gebundene Enzym ebenso aktiv ist wie das Enzym im freien Zustand, d.h. die spezifische Aktivität sowohl des gebundenen als auch des freien Enzyms sind gleich bzw. die relative Aktivität des Enzympräparats beträgt 100%. Bei Steigerung der Konzentration fällt dann jedoch von einer bestimmten Konzentration an die spezifische Aktivität rapide ab und die relative Aktivität wird kleiner als 100%, d.h. ein immer größerer Anteil des gebundenen Enzyms liegt in inaktiver Form vor.

Aus diesen Erkenntnissen heraus gibt die Erfindung die in den Ansprüchen niedergelegte Lehre zur Herstellung eines wasserunlöslichen Enzympräparats, die sowohl den hinsichtlich maximaler Aktivität optimalen Träger als auch die im Interesse einer maximalen Raum-Zeit-Ausbeute einerseits und der Vermeidung von Enzymverschwendung andererseits optimale Konzentration der Enzymlösung ermitteln läßt.

Bei Trägern, die aus einem $SiO_2$-Gel hergestellt sind, lässt sich gemäss Weiterbildung der Erfindung der optimale Träger erhalten, wenn man das Gel nach Einstellung eines Alkaligehalts, berechnet als $Na_2O$ und bezogen auf Trockensubstanz, von 0,1 bis 0,5 Gew.-%, und Trocknung, 5 bis 10 Stunden in einem wasserdampfhaltigen Luftstrom bei 400°C bis 850°C, vorzugsweise 570°C bis 750°C, glüht.

Zweckmässigerweise erfolgt die Trocknung in wasserdampfgesättigter Luft bei 180°C bis 200°C. Für das Glühen hat sich ein wasserdampfhaltiger Luftstrom mit einer relativen Feuchte von 40 bis 80% als vorteilhaft erwiesen.

Der so heregestellte Träger weist einen häufigsten Porendurchmesser von 175 bis 3.000 Å, vorzugsweise 250 bis 600 Å, optimalerweise etwa 340 Å, auf.

Das erfindungsgemässe Immobilisierungsverfahren ist für alle technisch und analytisch wichtigen Enzyme anwendbar, beispielsweise für Hydrolasen (z.B. Amylasen, Glycosidasen, Proteasen), Oxydoreductasen (Glucoseoxidase, Katalase), Isomerasen (Glucoseisomerase), Transferasen (Dextransucrase).

Fur den Fall, dass das Enzym Amyloglucosidase ist, welche im freien Zustand eine spezifische Aktivität von 10 bis 15 Einheiten/mg aufweist, wird das optimale Präparat dann erhalten, wenn man den optimalen Träger mit einer Lösung in Berührung bringt, die 25 bis 75 mg, vorzugsweise 50 mg, Amyloglucosidase pro Gramm Träger enthält.

Wird als Enzyme Glucoseisomerase eingesetzt, die im freien Zustand eine spezifischeAktivitat von 50—70 Einheiten/mg aufweist, erhält man das optimale Präparat, wenn der optimale Träger mit einer Lösung in Berührung gebracht wird, die 20—50 mg, vorzugsweise 25 mg, Glucoseisomerase pro Gramm Träger enthält.

Die nachfolgenden Beispiele dienen der Erläuterung der Erfindung.

## Beispiel 1

Zur Herstellung des Trägers 1 wurde ein aus Natriumsilikatlösung mit Schwefelsäure gefälltes $SiO_2$-Gel mit einem $Na_2O$-Gehalt von 0,3 Gew.-% bei 180°C in wasserdampfgesättigter Luft drei Stunden getrocknet. 1 kg dieses Materials wurde sechs Stunden bei 730°C in einem Luftstrom von 2 l/min, der einen relativen Feuchtigkeitsgehalt von 80% aufwies, geglüht. Nach dieser Behandlung hatte das $SiO_2$ einen häufigsten Porendurchmesser von 1400 Å. Der Träger 1 wurde durch Siebung in Fraktionen getrennt. Die weitere Präparation erfolgte mit der Fraktion 0,25 bis 0,5 mm.

150 g dieser Trägerfraktion wurde 8 Stunden mit 4 l einer 10%igen Lösung von $\gamma$-Aminopropyltriäthoxysilan in Benzol am Rückfluss gekocht und nach Abkühlung mit je 1000 ml Benzol und mit je 1000 ml Aceton dreimal gewaschen. Nach Abdampfen des Lösungsmittels bei Raumtemperatur im Vakuum wurde der Träger zweimal mit 0,05 m Phosphatpuffer (pH 7) und dreimal mit bidest. Wasser gewaschen. Die Trocknung erfolgte über $P_2O_5$ im Vakuum. Berechnet auf Basis des Mittelwertes der C- und N-Bestimmung enthielt der Träger 1 0,13 m Äq Silan/g.

10 g dieses Trägers wurden in 20 ml Lösung von 1 g Amyloglucosidase (Merck 1330) in 0,05 m Phosphatpuffer (pH 7) suspendiert. Die Aktivität der Amyloglucosidase betrug 11,75 U/mg, wobei als Aktivitätsmass U die Bildung von 1 $\mu$ Mol Glucose/min bei 25°C benutzt wurde. Die Suspension wurde 20 Minuten unter Vakuum gehalten, wieder belüftet und nach zwei Stunden nochmals für 20 Minuten evakuiert. Nach vier Stunden erfolgte die Trennung von Träger und Lösung durch Filtration, anschliessend dreimalige Waschung mit bidest. Wasser und schliesslich eine dreimalige Waschung mit 0,01 m Phosphatpuffer (pH 5). Die fertige Probe 1.1 wurde in Phosphatpuffer (pH 5) bei 4°C aufbewahrt. Die C-N-Analyse ergab einen Proteingehalt von 16,5 mg/g.

Zur Herstellung der Probe 1.2 wurden 10 g des silanisierten Trägers 1 in 20 ml Lösung von 0,5 g Amyloglucosidase (Merck 1330) in 0,05 m Phosphatpuffer (pH 7) suspendiert. Die weitere Verfahrensweise entsprach der Präparation der Probe 1.1. Die C-N-Analyse der fertigen Probe 1.2 ergab einen Proteingehalt von 9,0 mg/g.

## Beispiel 2

Zur Herstellung des Trägers 2 wurde ein aus Natriumsilikatlösung mit Schwefelsäure gefälltes $SiO_2$-Gel mit einem $Na_2O$-Gehalt von 0,3 Gew.-%, wie im Beispiel 1 beschrieben, getrocknet. 1 kg dieses Materials wurde sechs Stunden bei 680°C in einem Luftstrom von 2 l/min, der einen relativen Feuchtigkeitsgehalt von 80% aufwies, geglüht. Nach dieser Behandlung hatte das $SiO_2$ einen häufigsten Porendurchmesser von 340 Å. Der Träger 2 wurde durch Siebung in Fraktionen getrennt. Die weitere Präparation erfolgte mit der Fraktion 0,25 bis 0,5 mm.

150 g dieser Trägerfraktion wurde acht Stunden mit 4 l einer 10%igen Lösung von $\gamma$-Aminopropyltriäthoxysilan in Benzol entsprechend dem im Beispiel 1 angegebenen Verfahren behandelt.

Der Träger 2 enthielt, berechnet auf Basis des Mittelwertes der C- und N-Bestimmung, 0,19 m Äq Silan/g.

10 g dieses Trägers wurden in 20 ml Lösung von 1 g Amyloglucosidase (Merck 1330) in 0,05 m Phosphatpuffer (pH 7) suspendiert und, wie im Beispiel 1 beschrieben, präpariert. Die fertige Probe 2.1 wies nach der C-N-Analyse einen Proteingehalt von 30,8 mg/g auf.

Weitere 10 g des silanisierten Trägers 2 wurden in 20 ml Lösung von 0,5 g Amyloglucosidase (Merck 1330) in 0,05 m Phosphatpuffer (pH 7) suspendiert und, wie im Beispiel 1 (Probe 1.2) beschrieben, behandelt.

Die C-N-Analyse der fertigen Probe 2.2 ergab einen Proteingehalt von 17,4 mg/g.

## Beispiel 3

Zur Herstellung des Trägers 3 wurde ein aus Natriumsilikatlösung mit Schwefelsäure gefälltes $SiO_2$-Gel mit einem $Na_2O$-Gehalt von 0,3 Gew.-%, wie im Beispiel 1 beschrieben, getrocknet. 1 kg dieses Materials wurde sechs Stunden bei 640°C in einem Luftstrom von 2 l/min, der einen relativen Feuchtigkeitsgehalt von 60% aufwies, geglüht. Nach dieser Behandlung hatte das $SiO_2$ einen häufigsten Porendurchmesser von 180 Å. Der Träger 3 wurde durch Siebung in Fraktionen getrennt. Die weitere Präparation erfolgte mit der Fraktion 0,25 bis 0,50 mm.

150 g dieser Trägerfraktion wurde acht Stunden mit 4 l einer 10%igen Lösung von $\gamma$-Aminopropyltriäthoxysilan in Benzol entsprechend dem im Beispiel 1 angegebenen Verfahren behandelt.

Der Träger 3 enthielt, berechnet auf Basis des Mittelwertes der C- und N-Bestimmung 0,51 m Äq Silan/g.

10 g dieses Trägers wurden in 20 ml Lösung von 1 g Amyloglucosidase (Merck 1330) in 0,05 m Phosphatpuffer (pH 7) suspendiert und, wie im Beispiel 1 beschrieben, präpariert.

Die fertige Probe 3.1 wies nach der C-N-Analyse einen Proteingehalt von 26,2 mg/g auf.

Weitere 10 g des silanisierten Trägers 3 wurden in 20 ml Lösung von 0,5 g Amyloglucosidase (Merck 1330) in 0,05 m Phosphatpuffer (pH 7) suspendiert und, wie im Beispiel 1 (Probe 1.2) beschrieben, behandelt.

Die C-N-Analyse der fertigen Probe 3.2 ergab einen Proteingehalt von 12,7 mg/g.

### Beispiel 4

Un den Nachweis zu führen, dass das Kupplungsmittel auf die Aktivität des Präparates keinen Einfluss hat, wurde von dem Träger 2 (häufigster Porendurchmesser 340 Å) die Fraktion 0,25—0,5 mm ausgesiebt und davon 50 g in 500 ml 12,5%iger wäßriger Glutardialdehydlösung 5 Minuten bei Raumtemperatur gerührt. Anschliessend erfolgte ein Zusatz von 500 ml gesättigter $NH_4Cl$-Lösung. Nach vierstündigem Rühren bei Raumtemperatur wurde die Probe mit Wasser bis zur Chloridfreiheit gewaschen und über $P_2O_5$ im Vakuum getrocknet.

10 g dieses Trägers wurden in 20 ml Lösung von 1 g Amyloglucosidase (Merck 1330) in 0,05 m Phosphatpuffer (pH 7) suspendiert und wie im Beispiel 1 beschrieben, präpariert.

Die fertige Probe 4.1 wies nach der C-N-Analyse einen Proteingehalt von 29,8 mg/g auf.

Weitere 10 g des Trägers 2 wurden in 20 ml Lösung von 0,5 g Amyloglucosidase (Merck 1330) in 0,05 m Phosphatpuffer (pH 7) suspendiert und wie im Beispiel 1 (Probe 1.2) beschrieben, behandelt.

Die C-N-Analyse der fertigen Probe 4.2 ergab einen Proteingehalt von 17,9 mg.

### Beispiel 5

10 g des Trägers 1 (häufigster Porendurchmesser 1400 Å) wurden in 40 ml einer 0,05 m Phosphatpufferlösung (pH 7), die 0,5 g Glucoseisomerase (Y.Takasaki, Agr.Biol.Chem.33, Nr. 11, 1527—1534 (1969)) enthielt, suspendiert.

Die Reaktionszeit betrug bei Raumtemperatur 30 Minuten. Nach jeweils 10 Minuten wurde das Reaktionsgefäss evakuiert und nach Beendigung der Reaktion die Restlösung abgesaugt. Dann erfolgten 3 Waschungen mit Wasser und 0,05 m Phosphatpuffer (pH 7).

Die fertige Probe 5.1 wies nach der C-N-Analyse einen Proteingehalt von 4,8 mg/g auf.

Zur Herstellung der Probe 5.2 wurden weitere 10 g des Trägers 1 in 40 ml einer 0,05 m Phosphatpufferlösung (pH 7), die 0,25 g Glucoseisomerase enthielt, suspendiert. Die weitere Verfahrensweise entsprach der Präparation 5.1.

Die C-N-Analyse der fertigen Probe 5.2 ergab einen Proteingehalt von 2,0 mg/g.

### Beispiel 6

10 g des Trägers 2 (häufigster Porendurchmesser 340 Å) wurden in 40 ml einer 0,05 m Phosphatpufferlösung (pH 7), die 0,5 g Glucoseisomerase enthielt, suspendiert.

Die weitere Behandlung entsprach Beispiel 5. Die fertige Probe 6.1 wies nach der C-N-Analyse einen Proteingehalt von 22,0 mg/g auf.

Zur Herstellung der Probe 6.2 wurden weitere 10 g des Trägers 2 in 40 ml einer 0,05 m Phosphatpufferlösung (pH 7), die 0,25 g Glucoseisomerase enthielt, suspendiert. Die weitere Behandlung entsprach Beispiel 5. Die C-N-Analyse der fertigen Probe 6.2 ergab einen Proteingehalt von 10,2 mg/g.

### Beispiel 7

10 g des Trägers 3 (häufigster Porendurchmesser 180 Å) wurden in 40 ml einer 0,05 m Phosphatpufferlösung (pH 7), die 0,5 g Glucoseisomerase enthielt, suspendiert.

Die weitere Behandlung entsprach Beispiel 5. Die fertige Probe 7.1 wies nach der C-N-Analyse einen Proteingehalt von 11,2 mg/g auf.

Zur Herstellung der Probe 7.2 wurden weitere 10 g des Trägers 3 in 40 ml einer 0,05 m Phosphatpufferlösung (pH 7), die 0,25 g Glucoseisomerase enthielt, suspendiert. Die weitere Verfahrensweise entsprach der Präparation 5.1.

Die C-N-Analyse der fertigen Probe 7.2 ergab einen Proteingehalt von 5,1 mg/g.

### Beispiel 8

10 g des im Beispiel 4 genannten Trägers (häufigster Porendurchmesser 340 Å, behandelt mit wäßriger Glutardialdehydlösung) wurden in 40 ml einer 0,05 m Phosphatpufferlösung (pH 7), die 0,5 g Glucoseisomerase enthielt, suspendiert. Die weitere Behandlung entsprach Beispiel 5. Die fertige Probe 8.1 wies nach der C-N-Analyse einen Proteingehalt von 21,3 mg/g auf.

Zur Herstellung der Probe 8.2 wurden weitere 10 g des gleichen Trägers in 40 ml einer 0,05 m Phosphatpufferlösung (pH 7), die 0,25 g Glucoseisomerase enthielt, suspendiert. Die weitere Verfahrensweise entsprach der Präparation 5.1. Die C-N-Analyse der fertigen Probe 8.2 ergab einen Proteingehalt von 9,8 mg/g.

### Beispiel 9

Die Aktivität der in den Beispielen 1 bis 4 beschriebenen Präparate 1.1, 1.2; 2.1, 2.2; 3.1, 3.2; 4.1, 4.2 sowie die des zur Fixierung eingesetzten Enzyms (Amyloglucosidase, Merck 1330) wurde nach der Dinitrosalizylsäuremethode (vergl. Rick, W., Stegbauer, H.P. in: Bergmeyer, H.U. "Meth. d. Enzymatischen Analyse", Verlag Chemie 1970 S. 848 ff) bestimmt. Eine Aktivitätseinheit (U) entspricht der

# 0 000 028

Enzymmenge, die unter Inkubationsbedingungen 1 $\mu$ Äquivalent reduzierende Gruppen (berechnet als Glucose) pro Minute freisetzt.

*Inkubationsbedingungen*

2%ige Substratlösung (Zulkowsky-Stärke Merck 1257) in 0,1 m Acetatpuffer pH 5,0, 30 Minuten, 25°C.

Trägerfixierte Präparate wurden unter o.a. Bedingungen in einem 40 ml Reaktor bei einer Rührgeschwindigkeit von 600 $min^{-1}$ (Produktbildungsrate unabhängig von Rührgeschwindigkeit) suspendiert.

Der Proteingehalt der Präparate wurde auf Basis der Mittelwerte der C-N-Bestimmung ermittelt. Der häufigste Porendurchmesser der Träger wurde aus der Porenverteilung (gemessen mit Hochdruckporosimeter) bestimmt.

In der folgenden Tabelle 1 sind die Ergebnisse der Proben 1—4 zusammengestellt. Die verwendeten Kenngrössen werden wie folgt definiert:

| | | |
|---|---|---|
| Häufigster Porendurchmesser | D | (Å) |
| Enzymaufnahme | $c_E$ | (mg Enzym/g Träger) |
| Aktivität | U | (Units/g Probe) |
| Spez. Aktivität | $U_s = U/c_E$ (Units/mg Enzym) | |
| Spez. Aktivität i.freien Zustand | $U_{SF}$ (Units/mg Enzym) | |
| Relative Aktivität | $U_{rel} = 100 \cdot \dfrac{U_s}{U_{SF}}$ (%) | |

## TABELLE 1

### (Fixierte Amyloglucosidase)

| Probe | Häufigster Porendurchm. D | Enzymaufnahme $c_E$ | Aktivität | | | |
|---|---|---|---|---|---|---|
| | | | U | $U_s$ | $U_{rel}$ | $U_{SF}$ |
| 1.1 | 1400 | 16,5 | 98,4 | 5,96 | 51 | 11,75 |
| 1.2 | | 9,0 | 100,5 | 11,16 | 95 | |
| 2.1 | 340 | 30,8 | 208,8 | 6,77 | 58 | |
| 2.2 | | 17,4 | 203,9 | 11,71 | 100 | |
| 3.1 | 180 | 26,2 | 150,0 | 5,72 | 49 | |
| 3.2 | | 12,7 | 149,5 | 11,77 | 100 | |
| 4.1 | 340 | 29,8 | 199,7 | 6,70 | 57 | |
| 4.2 | | 17,9 | 209,4 | 11,70 | 100 | |

## Beispiel 10

Die Aktivität der in den Beispielen 5—8 beschriebenen Präparate 5.1, 5.2; 6.1, 6.2; 7.1, 7.2; 8.1, 8.2 sowie die der zur Fixierung eingesetzten Glucoseisomerase wurde nach der Takasaki-Methode (vgl. Y.Takasaki: Agr.Biol.Chem. Vol.30, Nr.12, 1247—1253, 1966 und Z.Dische u.E.Borenfreund: J.- Biol.Chem.192, 583, 1951) bestimmt. Eine Aktivitätseinheit (U) ist definiert als die Enzymmenge, die unter Inkubationsbedingungen 1 mg Fructose bildet.

## 0 000 028

*Inkubationsbedingungen*

| Temperatur | 65°C |
|---|---|
| Reaktionszeit | 1 h |
| Substrat | 0,1 m Glucose $xH_2O$ (Merck 8342) in 0,05 m Phosphatpuffer, pH 8,0 mit 0,0004 m $MGSO_4$ |

Die trägerfixierten Präparate wurden wie im Beispiel 9 beschrieben unter Standardbedingungen im Rührreaktor suspendiert.

Der Proteingehalt der Präparate wurde auf Basis der Mittelwerte der C-N-Bestimmung ermittelt. In der folgenden Tabelle 2 sind die Ergebnisse der Proben 5—8 zusammengestellt. Die Definition der verwendeten Kenngrössen ist im Beispiel 9 angegeben.

### TABELLE 2

#### (Fixierte Glucoseisomerase)

| Probe | Häufigster Porendurchm. $D$ | Enzymaufnahme $c_E$ | Activität | | | |
|---|---|---|---|---|---|---|
| | | | $U$ | $U_s$ | $U_{rel}$ | $U_{SF}$ |
| 5.1 | 1400 | 4,8 | 115,4 | 24,0 | 41 | 58,9 |
| 5.2 | | 2,0 | 117,3 | 58,7 | 99 | |
| 6.1 | 340 | 22,0 | 558,5 | 25,4 | 43 | |
| 6.2 | | 10,2 | 556,1 | 54,5 | 93 | |
| 7.1 | 180 | 11,2 | 291,0 | 26,0 | 44 | |
| 7.2 | | 5,1 | 292,3 | 57,3 | 97 | |
| 8.1 | 340 | 21,3 | 543,2 | 25,5 | 43 | |
| 8.2 | | 9,8 | 544,0 | 55,5 | 94 | |

*Die Ergebnisse zeigen:*

1. Die Aktivität $U$ der untersuchten Proben verläuft über ein Maximum, das vom häufigsten Porendurchmesser des Trägers abhängig ist.
2. Die spezifische Aktivität $U_s$ hängt von der Enzymaufnahme ab und erreicht bei einer bestimmten Enzymkonzentration $c_E$ den Wert der Aktivität des Enzyms im freien Zustand $U_{SF}$, so dass $U_{rel}=100$ wird. Wird diese Enzymmenge überschritten, sinkt die spezifische Aktivität ab, wobei das Produkt aus $U_s$ und $c_E$ konstant bleibt.
3. Die vom Träger aufgenommene Enzymmenge ist eine Funktion des häufigsten Porendurchmessers. Das System Enzym/Träger weist maximale Aktivität bei kleinstmöglichem Enzymeinsatz auf, wenn der optimale Träger 2 (häufigster Porendurchmesser 340 Å) 17,4 mg Amyloglucosidase/g bzw. 10,2 mg Glucoseisomerase/g aufgenommen hat (Proben 2.2 bzw. 6.2).
4. Enzymaufnahme und Aktivität der untersuchten Proben sind vom Kupplungsmittel unabhängig.

Da die Enzymkosten beträchtlich sind und mit dem geforderten Reinheitsgrad sehr stark ansteigen, für den wirtschaftlichen Einsatz also eine erhebliche Rolle spielen, ist mit dem erfindungsgemässen Verfahren erstmals die Möglichkeit geschaffen worden, auch teure Enzyme technisch anzuwenden, da es das Verfahren gestattet, bei Einsatz des erfindungsgemässen Trägers, die für maximale Aktivität benötigte Enzymmenge zu optimieren.

### Patentansprüche

1. Verfahren zur Herstellung eines wasserunlöslichen Enzympräparats, bei dem man einen anorganischen Träger, der funktionelle Gruppen zur kovalenten Bindung eines Enzyms aufweist, mit einer Lösung eines Enzyms in Berührung bringt, das Enzym mittels an sich bekannter Verfahren an den Träger bindet und die erhaltenen Enzympräparate isoliert, dadurch gekennzeichnet, daß man zunächst Träger, die sich in ihrem häufigsten Porendurchmesser unterscheiden, mit Enzymlösungen, die sich in ihrer Enzymkonzentration unterscheiden, in Berührung bringt, das Enzym an die Träger bindet, die Enzympräparate isoliert, deren Aktivität bestimmt und von den benutzten unterschiedlichen Trägern den Träger mit demjenigen häufigsten Porendurchmesser auswählt, der ungeachtet der an ihn gebundenen Enzymmenge das Präparat mit der höchsten Aktivität ergeben hat, und dann den ausgewählten Träger mit Enzymlösungen, die sich in ihrem Enzymgehalt unterscheiden, in Berührung bringt, das Enzym an den Träger bindet, die Enzympräparate isoliert, deren Aktivität und spezifische Aktivität bestimmt und von den benutzten unterschiedlichen Enzymlösungen diejenige auswählt, die das Präparat mit der höchsten Aktivität und einer spezifischen Aktivität nahe oder gleich der spezifischen Aktivität des Enzyms im freien Zustand ergeben hat.

2. Verfahren nach Anspruch 1, dadurch gekennzeichnet, daß man einen Träger aus einem $SiO_2$-Gel auswählt, das nach Einstellung eines Alkaligehalts, berechnet als $Na_2O$, auf 0,1 bis 0,5 Gew.-% und Trocknung 5—10 Stunden in einem wasserdampfhaltigen Luftstrom bei 400°C bis 850°C, vorzugsweise 570°C bis 750°C, geglüht ist.

3. Verfahren nach Anspruch 2, dadurch gekennzeichnet, daß die Trocknung in wasserdampfgesättigter Luft bei 180°C bis 200°C erfolgt.

4. Verfahren nach einem der Ansprüche 2 oder 3, dadurch gekennzeichnet, daß das Glühen in einem wasserdampfhaltigen Luftstrom mit einer relativen Feuchte von 40 bis 80% erfolgt.

5. Verfahren nach einem der Ansprüche 1 bis 4, dadurch gekennzeichnet, daß als Enzyme Amyloglucosidase verwendet wird.

6. Verfahren nach Anspruch 5, dadurch gekennzeichnet, daß bei Verwendung einer Amyloglucosidase mit einer spezifischen Aktivität im freien Zustand von etwa 10 bis 15 Einheiten/mg der Träger mit einer Lösung in Berührung gebracht wird, die 25 bis 75 mg, vorzugsweise 50 mg, Amyloglucosidase pro Gramm Träger enhält.

7. Verfahren nach einem der Ansprüche 1 bis 4, dadurch gekennzeichnet, daß als Enzym Glucoseisomerase verwendet wird.

8. Verfahren nach Anspruch 7, dadurch gekennzeichnet, daß bei Verwendung einer Glucoseisomerase mit einer spezifischen Aktivität im freien Zustand von etwa 50 bis 70 Einheiten/mg der Träger mit einer Lösung in Berührung gebracht wird, die 20 bis 50 mg, vorzugsweise 25 mg, Glucoseisomerase pro Gramm Träger enthält.

## Revendications

1. Procédé de fabrication d'une préparation enzymatique insoluble dans l'eau dans lequel un support inorganique qui comporte des groupes fonctionnels pouvant former une liaison covalente avec une enzyme est amenée en contact avec une solution d'enzyme, l'enzyme est liée au support par un procédé connu en soi et les préparations enzymatiques obtenues sont isolées, caractérisé en ce que l'on met d'abord des supports différant les uns des autres par leurs diamètres de pores les plus fréquents en contact avec des solutions d'enzyme différant les unes des autres par leur concentration en enzyme, en ce qu'on lie l'enzyme aux supports, en ce qu'on isole les préparations enzymatiques et détermine leur activité et en ce qu'on choisit parmi les divers supports utilisés celui dont le diamètre de pores le plus fréquent a donné la préparation ayant l'activité la plus élevée indépendamment de la quantité d'enzyme qui y est liée, et puis en ce qu'on met le support choisi en contact avec des solutions d'enzyme différant les unes des autres par leur concentration en enzyme, en ce qu'on lie l'enzyme au support, en ce qu'on isole les préparations enzymatiques et détermine leur activité et leur activité spécifique, et en ce qu'on choisit parmi les diverses solutions d'enzyme utilisées celle qui a donné la préparation ayant la plus grande activité et une activité spécifique égale ou presque égale à l'activité spécifique de l'enzyme à l'état libre.

2. Procédé suivant la revendication 1 caractérisé en ce qu'on choisit comme support un gel de $SiO_2$ qui, après réglage de la teneur en alcali, calculée en $Na_2O$, a une valeur de 0,1 à 0,5 en poids et séchage, est calciné pendant 5 à 10 heures dans un courant d'air contenant de la vapeur d'eau à une température de 400°C à 850°C, de préférence 570°C à 750°C.

3. Procédé selon la revendication 2 caractérisé en ce que le séchage dans de l'air saturé en vapeur d'eau s'effectue à une température de 180°C à 200°C.

4. Procédé selon l'une quelconque des revendications 2 ou 3, caractérisé en ce que la calcination s'effectue dans un courant d'air contenant de la vapeur d'eau présentant une humidité relative de 40 à 80%.

5. Procédé selon l'une quelconque des revendications 1 à 4 caractérisé en ce qu'on utilise l'amyloglucosidase comme enzyme.

6. Procédé selon la revendication 5 caractérisé en ce que, dans le cas de l'utilisation d'amyloglucosidase présentant une activité spécifique à l'état libre de l'ordre d'environ 10 à 15 unités/mg, le

# 0 000 028

support est amené en contact avec une solution qui contient 25 à 75 mg, de préférence 50 mg, d'amyloglucosidase par gramme de support.

7. Procédé selon l'une quelconque des revendications 1 à 4 caractérisé en ce qu'on utilise le glucoseisomérase comme enzyme.

8. Procédé selon la revendication 7 caractérisé en ce que, dans le cas de l'utilisation de glucoseisomérase présentant une activité spécifique à l'état libre de l'ordre d'environ 50 à 70 unités/mg, le support est amené en contact avec une solution qui contient 20 à 50 mg, de préférence 25 mg, de glucoseisomérase par gramme de support.

## Claims

1. A process for the production of a water-insoluble enzyme preparation, in which an inorganic support which possesses functional groups for covalent bonding of an enzyme is brought into contact with a solution of an enzyme, the enzyme is bonded to the support by processes which are in themselves known, and the resulting enzyme preparations are isolated, characterised in that, first, supports which differ from one another by their most frequent pore diameters are brought into contact with enzyme solutions which differ from one another in their enzyme concentrations, the enzyme is bonded to the support, the enzyme preparations are isolated, their activity is determined, and from amongst the different supports used, the support which has a particular most frequent pore diameter, and which, regardless of the amount of enzyme bonded to it, has given the preparation having the highest activity, is selected, and thereafter the selected support is brought into contact with enzyme solutions which differ from one another in their enzyme contents, the enzyme is bonded to the support, the enzyme preparations are isolated, their activity and specific activity are determined and, amongst the different enzyme solutions utilised, that which has given the preparation having the highest activity and having a specific activity close to or equal to the specific activity of the enzyme in the free state is selected.

2. A process according to Claim 1, characterised in that a support is selected from an $SiO_2$ gel which, after adjusting to an alkali content, calculated as $Na_2O$, of 0.1 to 0.5% by weight, and then drying, is calcined for 5 to 10 hours in a stream of air containing water vapour, at 400°C to 850°C, preferably 570°C to 750°C.

3. A process according to Claim 2, characterised in that the drying is carried out at 180°C to 200°C in air saturated with water vapour.

4. A process according to one of Claims 2 or 3, characterised in that the calcining is carried out in a stream of air containing water vapour, with a relative humidity of 40 to 80%.

5. A process according to one of Claims 1 to 4, characterised in that the enzyme used is amyloglucosidase.

6. A process according to Claim 5, characterised in that when using an amyloglucosidase having a specific activity in the free state of about 10 to 15 units/mg., the support is brought into contact with a solution which contains 25 to 75 mg., preferably 50 mg. of amyloglucosidase per gram of carrier.

7. A process according to one of Claims 1 to 4, characterised in that the enzyme used is glucoseisomerase.

8. A process according to Claim 7, characterised in that when using a glucoseisomerase having a specific activity in the free state of about 50 to 70 units/mg., the support is brought into contact with a solution which contains 20 to 50 mg., preferably 25 mg. of glucoseisomerase per gram of carrier.

9